# EUROPEAN PATENT APPLICATION

(11) **EP 1 413 327 A1**
(43) Date of publication of application: **28.04.2004**
(21) Application number: 02743818.3
(22) Date of filing: 04.07.2002
(51) Int. Cl.: A61M 29/02

(54) **STENT**

(30) Priority: 06.07.2001 JP 2001206665
(71) Applicant: TERUMO KABUSHIKI KAISHA, Tokyo 151-0072 (JP)
(72) Inventor: ISHII, Naoki, c/o Terumo kabushiki kaisha, Ashigarakami-gun, Kanagawa 259-0151 (JP); TOGAWA, Hideyuki, c/o Terumo kabushiki kaisha, Ashigarakami-gun, Kanaga 259-0151 (JP)
(74) Representative: Casalonga, Axel
(86) International application number: PCT/JP2002/006767
(87) International publication number: WO 2003/004089

(57) **Abstract**

A stent capable of stably loading a biologically/physiologically active substance, without causing its decomposition or deterioration, and permitting it to release itself slowly over a long period of time without being rapidly released in a short period, after being implanted in a lesion.

The stent (1) comprises a cylindrical stent main body (2) having an opening on each end and extending in axial direction between the two openings, and a sustained release coating formed on the surface of said stent main body from which a biologically/physiologically active substance is released, and wherein said sustained release coating is composed of a layer of a biologically/physiologically active substance (3) which covers the surface of said stent main body, and a polymer layer (4) which covers said layer of a biologically/physiologically active substance (3) on said layer of a biologically/physiologically active substance (3) , and said layer of a biologically/physiologically active substance (3) contains at least one kind of fat-soluble biologically/physiologically active substance, and said polymer layer (4) contains a polymer miscible with said biologically/physiologically active substance and fine particles (5) equal to or smaller than 5 µm in particle diameter dispersed in the polymer.

## Description

### Technical Field

The present invention relates to a stent which is designed to be implanted in a stenotic or occluded lesion that has occurred in such ducts of a living body as blood vessel, bile duct, trachea, esophagus, and urethra, thereby keeping the lesion open. More particularly, the present invention relates to a stent which slowly releases from its surface over a long period of time a biologically/physiologically active substance effective in preventing restenosis, thereby keeping open the lesion over a long period of time.

### Background Art

The following provides some background to the present invention with reference to angioplasty applied to ischemic heart disease.
The diffusion of westernized diets in Japan has steeply increased patients suffering from ischemic heart diseases (angina pectoris, cardiac infarction). Among new remedies against such heart diseases is percutaneous transluminal coronary angioplasty (PTCA), which is gaining general acceptance rapidly. It has matured technically and is now applied to more cases than before. PTCA was originally applied to a circumscribed lesion (with short lesion length) or a single vessel lesion (with the stenosis only at one vessel). Its application has been extended to a lesion which is distal, eccentric, or calcified, or a multivessel lesion (with the stenosis at two or more vessels).

PTCA is a procedure which consists of making a small incision in an artery of a patient's arm or leg, indwelling an introducer sheath in the incision, inserting a long tube called guide catheter into the blood vessel through the sheath, with a guide wire preceding, after the tube reaches the entrance of the coronary artery, withdrawing the guide wire, passing another guide wire and a balloon catheter through the guide catheter, advancing the balloon catheter under the X-ray radiography, with the guide wire preceding, to the lesion, namely the stenotic or occluded lesion in the coronary artery, and inflating the balloon once or more times at a prescribed pressure for 30 to 60 seconds. The result of this procedure is that the blood vessel at the lesion expands and remains open, thereby allowing more blood to flow in the blood vessel. The disadvantage of this procedure is that there is a possibility of the catheter damaging the vascular wall. In fact, it has been reported that restenosis occurs in a ratio of 30 to 40% by the growth of the vascular inner membrane which heals the damaged vascular wall.

Although no method for preventing restenosis has been firmly established yet, there is a promising one which employs such devices as stent and atherectomy catheter. The term "stent" used herein denotes a medical device in hollow cylindrical shape, usually made of metallic or polymeric material. It is used to cure various diseases resulting from stenosis or occlusion in blood vessels or other ducts. When in use, it expands the lesion, namely the stenotic or occluded lesion and is implanted there so as to keep open the blood vessel or duct. A variety of stents have been proposed so far. For example, one is a hollow cylindrical body having pores formed in the side wall thereof which is made of metallic or polymeric material, and another is a cylindrical body which is woven from metal wires or polymer fibers. The object of the implanted stent is to prevent or reduce the possibility of restenosis occurring after PTCA. However, it has turned out that any stent used alone cannot effectively prevent restenosis.

Nowadays, attempts are being made to reduce the possibility of restenosis in such a way that the stent is loaded with a biologically/physiologically active substance such as anticancer drug which locally and slowly releases itself in the implanted lesion. For example, JP 8-33718 A proposes a stent wherein a mixture composed of a therapeutical substance (a biologically/physiologically active substance) and a polymer is coated on the surface of the stent main body, and JP 9-99056 A proposes a stent wherein a layer of a bioactive material (a layer of a biologically/physiologically active substance) is formed on the surface of the stent main body, and a polymeric porous material layer is formed on the surface of the layer of the bioactive material.

Regrettably, the stent proposed in JP 8-33718 A has the disadvantage that the therapeutical substance (biologically/physiologically active substance) incorporated in the polymer is decomposed or deteriorated by chemical reactions between them. The problem with chemical instability of the biologically/physiologically active substance is explained below with reference to an example in which the polymer is polylactic acid. Because of its decomposability in a living body, polylactic acid permits the biologically/physiologically active substance to release itself efficiently. On the other hand, because of its tendency to liberate an acid upon decomposition, polylactic acid deteriorates the biologically/physiologically active substance which might have a weak acid resistance. Moreover, when a readily biodegradable polymer is selected, it permits the biologically/physiologically active substance to release itself in a short period (several days after implanted), resulting in making the stent unable to prevent restenosis sufficiently. In other words, the stent proposed in JP 8-33718 A, which is so designed as to prevent the biologically/physiologically active substance from decomposition and deterioration and to permit the biologically/physiologically active substance to release itself over a long period of time (several weeks to several months after implanted), has the disadvantage that the range of selection of the biologically/physiologically active substance is limited by combination with the polymer.

The stent proposed in JP 9-99056 A has no problems with the bioactive material (the biologically/physiologically active substance) being decomposed and deteriorated by the polymer because the biologically/physiologically active substance exists in a layer which is separated from the polymer layer. Nevertheless, it still has a drawback resulting from the fact that the layer of the biologically/physiologically active substance is covered with a porous polymer layer. In other words, the biologically/physiologically active substance remains exposed to the atmosphere surrounding the stent (the atmosphere outside the polymer layer) throughout the period from production to insertion into a living body, because the polymer layer has passages penetrating through it. Therefore, the stent of such structure would permit the biologically/physiologically active substance to release itself through the passages in the porous polymer layer before it is implanted at the lesion. Moreover, it is liable to cause the phenomenon that biologically/physiologically active substance is released rapidly in a short period (several days after implanted) after it has been implanted at the lesion, namely the initial burst. Thus, the above-mentioned stent presents difficulties in permitting the biologically/physiologically active substance to release itself slowly over a long period of time (several weeks to several months after implanted).

### Disclosure of Invention

It is an object of the present invention to provide a stent capable of stably loading a biologically/physiologically active substance, without causing its decomposition and deterioration, and permitting it to release itself slowly over a long period of time without being rapidly released in a short period, after implanted in a lesion.

The present invention to achieve the above-mentioned object includes the following aspects (1) to (12).
(1) A stent to be implanted in a duct of a living body, comprising:
   a cylindrical stent main body having an opening on each end and extending in axial direction between the two openings and
   a sustained release coating formed on the surface of said stent main body from which a biologically/physiologically active substance is released; wherein
   said sustained release coating composed of:
      a layer of a biologically/physiologically active substance which covers the surface of said stent main body; and
      a polymer layer which covers said layer of a biologically/physiologically active substance on said layer of a biologically/physiologically active substance,
      said layer of a biologically/physiologically active substance containing at least one kind of fat-soluble biologically/physiologically active substance,
      said polymer layer containing a polymer miscible with said biologically/physiologically active substance and fine particles equal to or smaller than 5 µm in particle diameter dispersed in the polymer.
(2) The stent as defined in (1) above wherein the stent main body is formed from a metallic material.
(3) The stent as defined in (1) above wherein the stent main body is formed from a polymeric material.
(4) The stent as defined in any of (1) to (3) above wherein the layer of a biologically/physiologically active substance is composed solely of a biologically/physiologically active substance.
(5) The stent as defined in any of (1) to (4) above wherein the biologically/physiologically active substance is any of antineoplastic agent, immunosuppressor, antibiotic, antirheumatic, antilipemic agent, ACE inhibitor, calcium antagonist, antiallergic agent, retinoid, antioxidant, and anti-inflammatory agent.
(6) The stent as defined in any of (1) to (5) above wherein the biologically/physiologically active substance is one which has a molecular weight not higher than 600.
(7) The stent as defined in any of (1) to (6) above wherein the polymer is a silicone polymer.
(8) The stent as defined in any of (1) to (7) above wherein the fine particles are those of a low-molecular-weight water-soluble salt having a molecular weight not higher than 1000.
(9) The stent as defined in (8) above wherein the low-molecular-weight salt is sodium chloride.
(10) The stent as defined in any of (1) to (7) above wherein the fine particles are those of a water-insoluble substance.
(11) The stent as defined in (10) above wherein the water-insoluble substance is a silica filler.
(12) The stent as defined in (10) above wherein the water-insoluble substance is polytetrafluoroethylene.

### Brief Description of Drawings

Fig. 1 is a side view showing one embodiment of the stent according to the present invention.
Fig. 2 is an enlarged transverse cross-sectional view taken along line A-A in Fig. 1.
Fig. 3 is a partially enlarged vertical cross-sectional view taken along line B-B in Fig. 1.
Fig. 4 is identical with Fig. 3 except that the fine particles in the polymer layer are dispersed in a different manner.
Fig. 5 is a schematic diagram showing the apparatus used in Example to measure the rate at which simvastatin passes through the polymer layer.

### Best Mode for Carrying out the Invention

The invention will be described in more detail with reference to the preferred embodiments in conjunction with the accompanying drawings.

The stent according to the present invention includes a stent main body and a sustained release coating which covers the surface of the stent main body.

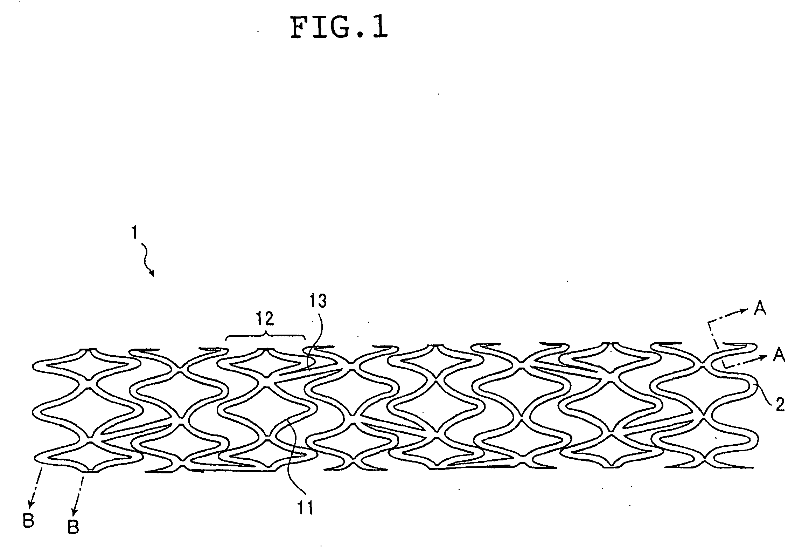

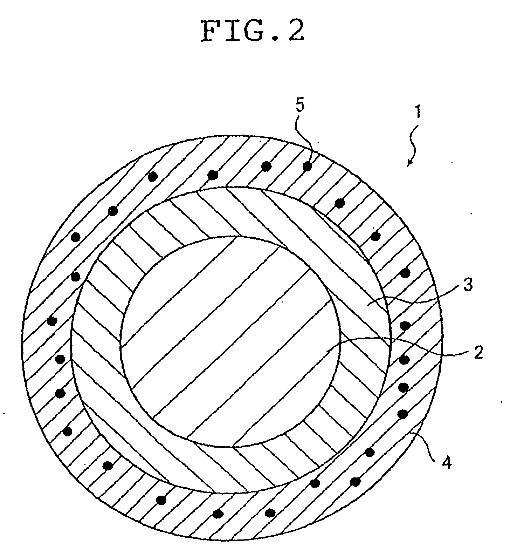

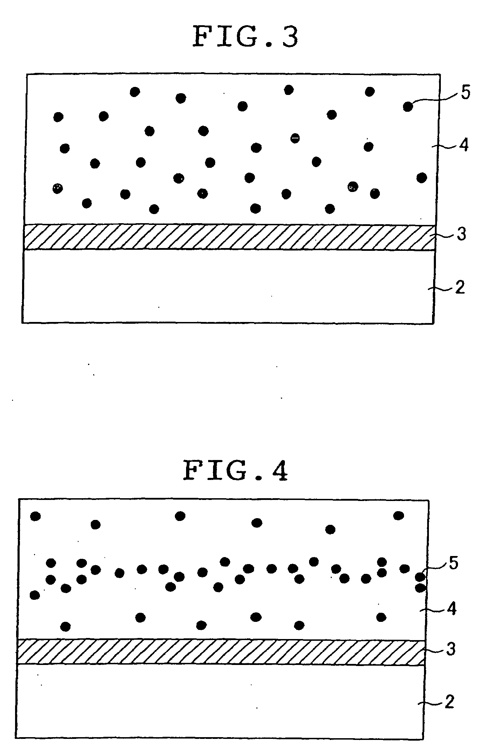

Fig. 1 is a side view showing one embodiment of the stent according to the present invention. Fig. 2 is an enlarged transverse cross-sectional view taken along line A-A in Fig. 1. Fig. 3 is a partially enlarged vertical cross-sectional view taken along line B-B in Fig. 1.

As Figs. 2 and 3 show, in the stent 1 according to the present invention, a layer 3 of biologically/physiologically active substance covers a fibrous member 2 on the surface of the fibrous member 2 constituting the stent main body , and a polymer layer 4 covers the layer 3 of biologically/physiologically active substance on the layer 3 of biologically/physiologically active substance. It is not always necessary that the layer 3 of biologically/physiologically active substance entirely covers the fibrous member 2 constituting the stent main body, but it is only necessary that the layer 3 of biologically/physiologically active substance partly covers the fibrous member 2 constituting the stent main body. Thus the stent may vary in the way the layer 3 of biologically/physiologically active substance covers the fibrous member 2. For example, the layer 3 of biologically/physiologically active substance may cover only that part of the fibrous member 2 which forms the outer surface of the stent main body which is cylindrical body. Conversely, the layer 3 of biologically/physiologically active substance may cover only that part of the fibrous member 2 which forms the inner surface of the stent main body. On the other hand, the layer 3 of biologically/physiologically active substance should always be covered with the polymer layer 4.

A more detailed mention is made below of the components constituting the stent 1 according to the present invention.

The stent main body is an cylindrical body having an opening on each end and extending in axial direction between these openings. The wall of the cylindrical body has a large number of holes passing across it between the outer surface and the inner surface. The cylindrical body is constructed such that it expands or contracts in its radial direction as the holes deform. It retains its shape while it is implanted in a duct of a living body such as blood vessel and bile duct.

The stent main body shown in Fig. 1 is constituted of an elastic fibrous material 2 and composed of basic units 11 having a rhombic hole. A plurality of the basic units 11 arranged and connected to one another in the circumferential direction form an annular unit 12. One annular unit 12 joins to its adjacent ones through a linear elastic material 13. Thus a plurality of annular units 12 are continuously arranged in the axial direction with partial connection to one another. The stent main body (stent) 1 constructed in this manner forms an cylindrical body having an opening on each end and extending in axial direction between these openings. Moreover, the stent main body (stent) 1 has rhombic holes, so that it expands or contracts in its radial direction as the holes deform.

The stent main body according to the present invention is not limited to that shown in Fig. 1, however, it may include the one which is an cylindrical body having an opening on each end and extending in axial direction between these openings and has a large number of holes passing across its wall between the outer surface and the inner surface, so that the cylindrical body expands or contracts in its radial direction as the holes deform.

Examples of the stent main body that expands or contract in its radial direction are found in the following disclosures.

JP 9-215753 A and JP 7-529 A propose a stent main body in a cylindrical shape consisting of a plurality of coils of elastic wires joined together. Gaps between the elastic wires function as holes.

JP 8-502428 A and JP 7-500272 A propose a stent main body in a cylindrical shape consisting of zigzag elastic wires joined together. Gaps between the elastic wires function as holes.

JP 2000-501328 and JP 11-221288 A propose a stent main body in a cylindrical shape consisting of winding elastic wires in a helical form. Gaps between the elastic wires function as holes.

JP 10-503676 proposes a stent main body in mesh structure having meanderingly arranged holes unlike the stent main body shown in Fig. 1.

JP 8-507243 proposes a stent main body in a cylindrical shape formed with a coiled flat member. Gaps between adjacent coils function as holes.

JP 4-68939 B propose a variation of stent main body including two cylindrical bodies, one being formed with a helically wound elastic flat member and having gaps functioning as holes between adjacent coils, and the other being formed with braded elastic wires and having gap functioning as holes between elastic wires. The stent main body of the present invention may be in any form like coiled leaf spring, multiple coil, odd-shape tube, and so on. In addition, the stent main body of the present invention may also be something similar to the one shown in Figs. 2(a) and 2(b) in JP 4-68939 B. It is a cylindrical body formed with a coiled elastic flat member. The cylindrical body has no holes in its side wall but expands and contracts in its radial direction. Citations from the above-mentioned literature and patent application constitute a part of this specification.

No specific restrictions are imposed on the method of expanding the stent main body which has been implanted in a lesion. The stent main body may be of self-expansive type, that is, it expands in its radial direction by its own restoring force when it is released from the force to fold it small. Alternatively, the stent main body may be of ballon-expandable type, that is, it is expanded in its radial direction by an external force as the balloon placed therein is inflated.

The stent main body may be formed from a polymeric material, metallic material, carbon fiber, ceramics, or the like. These materials are not specifically restricted so long as they have a certain degree of stiffness and elasticity. However, those which are compatible with a living body are preferable.

Typical examples of the materials are listed below.

Polymeric materials: polyolefins such as polyethylene and polypropylene, polyesters such as polyethylene terephthalate, cellulosic polymers such as cellulose acetate and cellulose nitrate, and fluoroplastics such as poly-tetrafluoroethylene, tetrafluoroethylene-ethylene copolymer.

Metallic materials: stainless steel, tantalum, titanium, nickel-titanium alloy, tantalum-titanium alloy, nickel-aluminum alloy, Inconel, gold, platinum, iridium, tungsten, and cobalt alloy. In some variation of stainless steel, SUS316L is desirable because of its outstanding corrosion resistance.

The stent main body may be formed as desired from any of the above-mentioned materials which is selected according to the lesion to which the stent is applied or the means by which the stent is expanded. A stent formed from a metallic material ensures being implanted in the lesion on account of the high strength of metal. A stent formed from a polymeric material permits easy delivery to the lesion on account of the good flexibility of polymer.

A stent of self-expansive type should preferably be formed from a highly elastic alloy such as titanium-nickel alloy so that it has a sufficient restoring force. A stent of balloon-expandable type should preferably be formed from stainless steel so that it will not return to its original shape after expansion.

A stent made of carbon fiber is desirable because of its high strength, good flexibility, and high safety in a living body.

The size of the stent main body should be determined according to the lesion to which the stent is applied. It should preferably be in the range of 1.0 to 3.0 mm in outside diameter and in the range of 5 to 50 mm in length before expansion, if the stent is to be applied to the coronary artery.

In the case of a stent main body made of a fibrous member, the widthwise length of the fibrous member constituting holes should be in the range of 0.01 to 0.5 mm, preferably in the range of 0.05 to 0.2 mm.

The method of producing the stent main body is not specifically restricted; an ordinary one may be employed according to the structure and material of the stent.

In the stent according to the present invention, a layer 3 of biologically/physiologically active substance covers the surface of the fibrous member 2 constituting the above-mentioned stent main body. In the case of a stent main body formed from other constituent besides the fibrous member, for example, a sheet member, a layer 3 of biologically/physiologically active substance covers the surface of the constituent like the sheet member.

The layer 3 of biologically/physiologically active substance should contain at least one kind of fat-soluble biologically/physiologically active substance, which is not specifically restricted so long as it is effective in preventing restenosis while the stent is implanted in the lesion. It includes, for example, antineoplastic agent, immunosuppressor, antibiotics, antilipemic agent, ACE inhibitor, calcium antagonist, antiallergic agent, retinoid, antioxidant, and anti-inflammatory agent.

Preferred antineoplastic agents include, for example, irinotecan hydrochloride, paclitaxel, docetaxel hydrate, and methotrexate.

Preferred immunosuppressors include, for example, sirolimus, tacrolimus hydrate, azathioprine, ciclosporin, and mycophenolate mofetil.

Preferred antibiotics include, for example, mitomycin C, doxorubicin hydrochloride, actinomycin D, idarubicin hydrochloride, and pirarubicin hydrochloride.

Preferred antilipemic agents include, for example, probucol and HMG-CoA reductase inhibitor such as cerivastatin, simvastatin, and lobastatin.

Preferred ACE inhibitors include, for example, trandolapril, cilazapril, temocapril hydrochloride, delapril hydrochloride, and enalapril maleate.

Preferred calcium antagonists include, for example, nilvadipine, benidipin hydrochloride, and nisoldipine.

Preferred antiallergic agents include, for example, tranilast.

Preferred retinoids include, for example, all-trans retinoic acid.

Preferred antioxidants include, for example, epigallocatechin gallate.

Preferred anti-inflammatory agents include, for example, aspirin and steroids such as dexamethazone and prednisolone.

The biologically/physiologically active substance should desirably be a low-molecular-weight one which has a molecular weight not higher than 600. Such a biologically/physiologically active substance passes through the polymer layer at a relatively high rate if fine particles are not dispersed in the polymer layer. However, it releases itself slowly if fine particles are dispersed in the polymer layer.

Of the biologically/physiologically active substances illustrated above, typical examples having a molecular weight not higher than 600 include cerivastatin, simvastatin, and lobastatin.

The layer of biologically/physiologically active substance may contain only one kind of the biologically/physiologically active substance illustrated above, or alternatively ,more than one kind of the biologically/physiologically active substances of the combination adequately selected from the biologically/physiologically active substances illustrated above according to need.

The layer 3 of biologically/physiologically active substance is formed on the surface of the fibrous member 2 constituting the stent main body, as mentioned above. The method for this step is not specifically restricted so long as it permits the layer 3 of biologically/physiologically active substance to be formed uniformly on the surface of the fibrous member 2 constituting the stent main body. One possible way is to apply the biologically/physiologically active substance in a molten state to the surface of the fibrous member 2 constituting the stent main body. Another way is to dip the stent main body into the biologically/physiologically active substance in a molten state, followed by pulling out and cooling. It is also possible to coat the fibrous member constituting the stent main body with the layer of the biologically/physiologically active substance by dissolving the biologically/physiologically active substance in an adequate solvent and then dipping the stent main body into the resulting solution, followed by pulling up and drying for solvent removal, or spraying the stent main body with the resulting solution, followed by drying for solvent removal.

In the case where the biologically/physiologically active substance alone does not form its layer on the surface of the fibrous member 2 constituting the stent main body due to its insufficient adhering force, it is desirable to add an additional component for imparting adhesion to the solution. Examples of such an additional component include low-molecular-weight higher fatty acid with a molecular weight not higher than 1000 such as fish oil and vegetable oil, and fat-soluble vitamins such as vitamin A and vitamin E. If the additional component has a melting point low enough not to harm the biologically/physiologically active substance, then it is possible to heat the coating film above the melting point of the mixture so that the layer of the biologically/physiologically active substance firmly adheres to the surface of the fibrous member 2 constituting the stent main body.

The layer of the biologically/physiologically active substance can be formed most easily and simply by dipping or spraying if there exists an adequate solvent for the biologically/physiologically active substance and the resulting solution alone can form a layer on the surface of the fibrous member 2 constituting the stent main body.

The biologically/physiologically active substance may vary in amount in its layer depending on the shape and size of the stent. An desirable amount ranges from 100 to 1,000 µg/cm2. An adequate amount should be established so that the biologically/physiologically active substance fully produces its effect without adversely affecting the performance of the stent main body (such as ability to be delivered to the lesion and freedom from stimulant action on vascular walls).

In the stent according to the present invention, a polymer layer 4 covers the layer 3 of the biologically/physiologically active substance. The polymer layer 4 is composed of a polymer miscible with the biologically/physiologically active substance, and fine particles equal to or smaller than 5 µm in particle diameter are dispersed in the polymer layer 4.

Being miscible with the biologically/physiologically active substance, the polymer constituting the polymer layer 4 dissolves the biologically/physiologically active substance at the interface between the polymer layer 4 and the layer 3 of the biologically/physiologically active substance. The dissolved biologically/physiologically active substance passes through the polymer layer 4 and releases itself to the outside of the sustained release coating. If the polymer layer 4 is composed only of a polymer miscible with the biologically/physiologically active substance, it would permit the biologically/physiologically active substance to pass through the polymer layer 4 and release itself to the outside of the polymer layer 4 rapidly in a short period . However, this is not the case with the stent of the present invention, in which the polymer layer 4 contains fine particles 5 equal to or smaller than 5 µm in particle diameter dispersed therein which control the rate at which the biologically/physiologically active substance releases itself. In other words, the fine particles 5 dispersed in the polymer layer 4 function as obstacles to the biologically/physiologically active substance passing through the polymer layer 4, thereby retarding and controlling the passage of the biologically/physiologically active substance. As the result, the biologically/physiologically active substance releases itself from the polymer layer 4 slowly over a long period of time without being rapidly released in a short period.

The polymer from which the polymer layer 4 is formed is not specifically restricted so long as it is misclible with the biologically/physiologically active substance forming the layer 3 of the biologically/physiologically active substance. Therefore, it should be selected according to the biologically/physiologically active substance used. It is usually fat-soluble because the biologically/physiologically active substance is fat-soluble.

The polymer for the polymer layer 4 should have high stability in a living body and low stimulation to tissues in view of the fact that it is used for the stent implanted in a duct of a living body. Examples of such a polymer include silicone polymer such as silicone elastomer, cellulosic plastics, polyurethane, polyester, vinyl polymer, acrylic polymer, and thermoplastic elastomer. Of these examples, silicone elastomer is most desirable.

A silicone elastomer denotes any elastomer composition composed mainly of straight-chain or branched-chain dialkylpolysiloxane. The dialkylpolysiloxane may have its alkyl groups partly replaced by vinyl groups, amino groups, hydrogen atoms, chlorine atoms, and so on, although more than 90% of alkyl groups are methyl groups. The silicone elastomer should have a viscosity not lower than 500 cps before curing and a durometer hardness (Shore A) of 20 to 80 after curing. Incidentally, a silicone elastomer of two-pack system is preferable because of its good workability in forming the polymer layer 4. Silicone elastomers meeting the above-mentioned conditions are commercially available under a trade name of, for example, "Silastic" (Dow Corning Corporation) and "Silicone Elastomer MED-4211" (Nusil).

The above-mentioned polymer may contain additional components such as plasticizer and filler in an amount not harmful to the effect of the present invention.

No specific restrictions are imposed on the method for covering the layer 3 of biologically/physiologically active substance with the polymer layer 4 and the method for dispersing the fine particles 5 in the polymer layer 4, so long as the layer 3 of biologically/physiologically active substance is covered completely with the polymer layer 4 and the fine particles 5 are uniformly or unevenly as mentioned later dispersed in the polymer layer 4. One method may consist of dissolving a polymer and the fine particles in a solvent and dipping the stent main body covered with the layer of biologically/physiologically active substance in the resulting solution, followed by drying. The other method may consist of spraying the resulting solution onto the fibrous member constituting the stent main body, the fibrous member being previously covered with the layer of biologically/physiologically active substance, followed by drying.

As in the case of the layer 3 of biologically/physiologically active substance, the thickness of the polymer layer 4 should be established in an adequate range which is not harmful to the performance of the stent main body 2 such as easy delivery to the lesion and non stimulus to the vascular wall. The thickness of the polymer layer 4 should preferably be in the range of 1 to 75 µm, more preferably in the range of 10 to 50 µm, and most desirably in the range of 20 to 30 µm. With a thickness smaller than 1 µm, the polymer layer 4 does not completely cover the layer 3 of biologically/physiologically active substance because the diameter of the fine particles contained therein are relatively larger than the thickness of the polymer layer 4. Conversely, with a thickness larger than 75 µm, the polymer layer makes the outside diameter of the stent itself excessively large, thereby reducing the ease with which the stent is delivered to the lesion.

The fine particles 5 to be dispersed in the polymer layer 4 should preferably be those of water-soluble substance, particularly a low-molecular-weight water-soluble salt having a molecular weight not higher than 1000. The term "low-molecular-weight water-soluble salt" denotes an ionic compound formed by neutralization of an acid and a base. The fine particles 5 of the water-soluble substance absorb water or water vapor which has entered the polymer layer 4, thereby forming water particles scattered in the polymer layer 4. The water or water vapor which enters the polymer layer 4 is the one which exists in a duct of a living body while the stent 1 is implanted in the lesion. The water vapor is formed as the result of evaporation of humor such as blood present in the duct of the living body. The water particles resulting from the water-soluble substance absorbing water or water vapor function as an obstacle to the passage of the fat-soluble biologically/physiologically active substance through the polymer layer 4, thereby retarding and controlling the rate at which the biologically/physiologically active substance releases itself from the sustained release coating.

For the reason mentioned above, in the case where the fine particles 5 are those of a water-soluble salt, the polymer layer 4 should be permeable to water or water vapor.

According to the present invention, the fine particles 5 to be dispersed in the polymer layer 4 should preferably be those of an inactive substance composed of an inorganic substance or an organic substance insoluble in water and fat. Such an inactive substance is hardly decomposable and inactive to a living body. When dispersed in the polymer layer 4, it functions as an obstacle to the passage of the biologically/physiologically active substance through the polymer layer 4, thereby controlling the rate at which the biologically/physiologically active substance releases itself. Examples of such an inactive substance include silicon compounds such as silica filler, carbon allotrope such as graphite, carbon black, fullerene, and carbon nanotube, and fluoroplastics such as polytetrafluoroethylene (PTFE). Of these examples, silica filler and PTFE are desirable because of their good appearance and low price.

These inactive substances may undergo surface treatment without any adverse effect on their inactiveness. The surface treatment improves dispersibility into the polymer layer 4.

The concentration of the fine particles 5 should preferably be in the range of 0.0001 to 1 mass% of the polymer constituting the polymer layer 4 in the case where they are those of a low-molecular-weight water-soluble salt such as sodium chloride. On the other hand, the concentration of the fine particles 5 should preferably be in the range of 10 to 100 mass% of the polymer constituting the polymer layer 4 in the case where they are those of an inactive substance.

The fine particles 5 used more than the above-mentioned range might greatly impair the mechanical properties of the polymer layer 4. Alternatively, the fine particles 5 used less than the above-mentioned range do not fully function as an obstacle in the polymer layer 4 and hence they fail to control the rate at which the biologically/physiologically active substance diffuses from the biologically/physiologically active substance layer 3 into the polymer layer 4 and releases itself from the stent.

In the stent of the present invention, no specific restrictions are imposed on how the fine particles 5 are dispersed in the polymer layer 4. It is not always necessary that the fine particles 5 should be uniformly dispersed in the polymer layer 4 as shown in Fig. 3. The fine particles 5 may be dispersed unevenly in the polymer layer 4 as shown in Fig. 4. Fig. 4 is the same partial cross-sectional views as fig. 3. Fig. 4 shows that the fine particles 5 are not uniformly dispersed in the polymer layer 4 and there is a layer-like part with a high concentration of the fine particles 5 held between layer-like parts with a low concentration of the fine particles 5. The uneven distribution of the fine particles 5 in the polymer layer 4 permits the high-concentration part to prevent the passage of the biologically/physiologically active substance. Nevertheless, the fine particles 5 dispersed in this manner do not impair the mechanical properties of the polymer layer 4 unlike the case in which the concentration of the fine particles is high throughout the polymer layer 4, because the concentration of the fine particles 5 is not necessarily high throughout the polymer layer 4. In the case of uneven distribution as mentioned above, it is possible to increase the concentration of the fine particles partially in excess of 100 mass% without any adverse effect on the mechanical properties of the polymer layer 4.

The fine particles 5 to be added to the polymer layer 4 are not specifically restricted in particle diameter so long as they are small enough not to roughen the surface of the polymer layer 4. The particle diameter should be in the range of 0.01 to 5 µm, preferably in the range of 0.03 to 3 µm, most desirably in the range of 0.05 to 1 µm, in view of the thickness of the polymer layer 4.

Incidentally, the polymer for the polymer layer 4 and the fine particles 5 are not specifically restricted in kind so long as the fine particles 5 are dispersible in the polymer layer 4 and are capable of functioning as an obstacle in the polymer layer 4. A desirable combination is as follows.

In the case of a water-soluble substance as the fine particles 5, sodium chloride having a particle diameter of 0.01 to 1 µm should be dispersed in the silicone elastomer mentioned above in an amount of 0.0001 to 1 mass%.

In the case of an inactive substance as the fine particles 5, silica filler or PTFE having a particle diameter of 0.01 to 1 µm should be dispersed in the silicone elastomer mentioned above in an amount of 10 to 100 mass%.

### Examples

The invention will be described in more detail with reference to the following examples, which are not intended to restrict the scope thereof.

### (1) Test for permeation of the biologically/physiologically active substance through the polymer film

### Example 1

A polymer film having a thickness of 30 µm was prepared by casting in a petri dish from a hexane solution of two-pack silicone elastomer (10 wt% in concentration) incorporated with PTFE fine particles (not larger than 1 µm in particle diameter). The two-pack silicone elastomer is "Q7-4840 Silastic" (from Dow Corning Corporation) consisting of solution-A and solution-B in equal quantities. The amount of the PTFE is 30 wt% on the total amount of the polymer in the silicone elastomer and the PTFE fine particles.

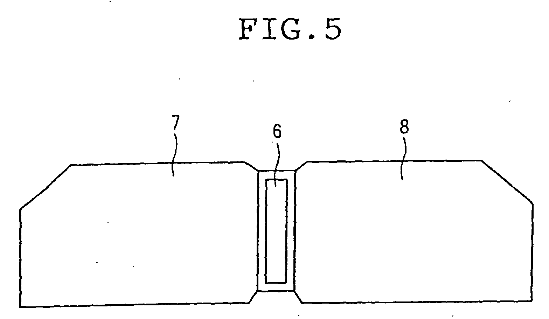

The thus obtained polymer film was placed at the intermediate part 6 of two chambers 7 and 8 of the apparatus shown in Fig. 5. One chamber 7 of the apparatus was filled with an aqueous solution of simvastatin (SVS) which had been filtered off through a 0.45 µm thick membrane filter made by Advantec. The aqueous solution contains 50 µg/ml conc. of SVS and 2 wt% of surfactant ("Tween 20" from Nikko Chemical Co,. Ltd.). The other chamber 8 was filled with reverse osmosis (RO) water containing 2 wt% of surfactant ("Tween 20"). The apparatus was allowed to stand in a thermostat at 37 °C in order to measure the rate (cm/sec) at which SVS permeates through the polymer film. The rate of permeation was measured in the following manner. The solution in the chamber 8 is sampled (1 ml each) at prescribed time intervals: Each sample is measured for absorbance at a wavelength of 238 nm by using a spectrophotometer (UV-2400PC made by Shimadzu Corporation). The readings of absorbance are converted into the amount of permeation of SVS by using a previously prepared calibration curve. The amount of permeation is further converted into the amount per unit time and unit area of the polymer film. The results of the test are shown in Table 1.

### Example 2

A polymer film having a thickness of 30 µm was prepared by casting in a petri dish from a hexane solution of two-pack silicone elastomer (10 wt% in consentration) incorporated with sodium chloride fine particles (not larger than 1 µm in particle diameter). The two-pack silicone elastomer is "Q7-4840 Silastic" (from Dow Corning Corporation) consisting of solution-A and solution-B in equal quantities. The amount of sodium chloride is 0.25 wt% on the total amount of the polymer in the silicone elastomer and the sodium chloride fine particles. The thus obtained polymer film was measured for the rate of permeation of SVS in the same way as in Example 1. The results of the test are shown in Table 1.

### Comparative Example 1

A polymer film having a thickness of 30µm was prepared by casting in a petri dish from a hexane solution of two-pack silicone elastomer (10 wt% in concentration) which is not incorporated with PTFE or sodium chloride fine particles. The two-pack silicone elastomer is "Q7-4840 Silastic" (from Dow Corning Corporation) consisting of solution-A and solution-B in equal quantities. The thus obtained polymer film was measured for the rate of permeation of SVS in the same way as in Example 1. The results of the test are shown in Table 1.

**Table 1**

| Rate of permeation of SVS through polymer film (cm/sec) | |
|---|---|
| Example 1 | 2.713E-05 |
| Example 2 | 2.244E-05 |
| Comparative Example 1 | 3.697E-05 |

It is noted from Table 1 that the polymer film in Examples 1 and 2 which contains PTFE or sodium chloride fine particles reduces the rate of permeation of SVS through it.

### (2) Measurement of the rate of sustained release of biologically/physiologically active substance

### Example 3

A stent sample was prepared in the following manner from a stent main body which is in a cylindrical shape (1.8 mm in outside diameter and 15 mm long, having approximately rhombic holes) and is composed of fibrous members (0.1 mm in diameter) of stainless steel (SUS 316L), as shown in Fig. 1. This stent main body was sprayed with a hexane solution of SVS (5 wt% in concentration) by using a hand spray (HP-C made by Iwata Corporation). It was confirmed that the surface of the fibrous members constituting the stent main body was coated with about 200 µg of SVS. Upon complete solvent (hexane) removal by evaporation, there was formed a layer of SVS (biologically/physiologically active substance) on the surface of the fibrous members constituting the stent main body.

The coated stent main body was sprayed with a hexane solution of two-pack silicone elastomer incorporated (2 wt% in concentration) with PTFE fine particles (not larger than 1 µm in particle diameter). The two-pack silicone elastomer is "Q7-4840 Silastic" (from Dow Corning Corporation) consisting of solution-A and solution-B in equal quantities. The solution of two-pack silicone elastomer is composed of 1 g each of solution-A and solution-B and 98 g of hexane. The amount of PTFE fine particles is 30 wt% on the total amount of the polymer in the silicone elastomer and the PTFE fine particles. The solution was applied to the stent main body by using a hand spray such that the layer of biologically/physiologically active substance is completely covered with the polymer layer. The polymer layer was cured by heating at 80 °C for 1 hour in an oven. The resulting polymer layer was found to have an average thickness of about 30 µm. The thus obtained stent sample was measured for the rate at which SVS releases itself.

Measurement was carried out in the following manner. The stent sample is immersed in RO water containing 2 wt% of surfactant (Tween 20) which is held in a thermostat at 37 °C. The RO water is sampled (1 ml each) at prescribed time intervals, and the amount of SVS in each sample is determined in the same way as in Example 1 and the rate of sustained release of SVS is calculated. The results are shown in Table 2. The values in Table 2 denote the ratio (%) of the amount of SVS released to the amount of SVS applied to the fibrous members constituting the stent main body.

### Example 4

The same stent main body as used in Example 3 was sprayed with a hexane solution of SVS (5 wt% in concentration) by using a hand spray. It was confirmed that the surface of the fibrous members constituting the stent main body was coated with about 200 µg of SVS. Upon complete solvent (hexane) removal by evaporation, there was formed a layer of SVS. The coated stent main body was sprayed with a hexane solution of two-pack silicone elastomer (2 wt% in concentration) incorporated with sodium chloride fine particles (not larger than 1 µm in particle diameter). The two-pack silicone elastomer is "Q7-4840 Silastic" (from Dow Corning Corporation) consisting of solution-A and solution-B in equal quantities. The solution of two-pack silicone elastomer is composed of 1 g each of solution-A and solution-B and 98 g of hexane. The amount of sodium chloride fine particles is 0.25 wt% on the total amount of the polymer in the silicone elastomer and the sodium chloride fine particles. The solution was applied to the stent main body by using a hand spray such that the layer of biologically/physiologically active substance is completely covered with the polymer layer. The polymer layer was cured by heating at 80 °C for 1 hour in an oven. The resulting polymer layer was found to have an average thickness of about 30 µm. The thus obtained stent sample was measured for the rate at which SVS releases itself.

Measurement was carried out in the same way as in Example 3. The results are shown in Table 2. The values in Table 2 denote the ratio (%) of the amount of SVS released to the amount of SVS applied to the fibrous members constituting the stent main body.

### Comparative Example 2

The same stent main body as used in Example 3 was sprayed with a hexane solution of SVS (5 wt% in concentration) by using a hand spray. It was confirmed that the surface of the fibrous members constituting the stent main body was coated with about 200 µg of SVS. Upon complete solvent (hexane) removal by evaporation, there was formed a layer of SVS. The coated stent main body was sprayed with a hexane solution of two-pack silicone elastomer (2 wt% in concentration) which is not incorporated with sodium chloride or PTFE fine particles. The two-pack silicone elastomer is "Q7-4840 Silastic" (from Dow Corning Corporation) consisting of solution-A and solution-B in equal quantities. The solution of two-pack silicone elastomer is composed of 1 g each of solution-A and solution-B and 98 g of hexane. The solution was applied to the stent main body by using a hand spray such that the layer of biologically/physiologically active substance is completely covered with the polymer layer. The polymer layer was cured by heating at 80 °C for 1 hour in an oven. The resulting polymer layer was found to have an average thickness of about 30 µm. The thus obtained stent sample was measured for the rate at which SVS releases itself.

Measurement was carried out in the same way as in Example 3. The results are shown in Table 2. The values in Table 2 denote the ratio (%) of the amount of SVS released to the amount of SVS applied to the fibrous members constituting the stent main body.

**Table 2**

| Ratio (%) of sustained release of SVS | | | | |
|---|---|---|---|---|
| | 0 h | 23 h | 72 h | 240 h |
| Example 3 | 0.0 | 4.3 | 11.1 | 36.4 |
| Example 4 | 0.0 | 2.9 | 9.7 | 27.4 |
| Comparative Example 2 | 0.0 | 4.5 | 13.9 | 56.7 |

Values in Table 2 denote the ratio (%) ot the amount of SVS released to the amount of SVS applied to the fibrous members constituting the stent main body.

It is noted from Table 2 that the stent samples in which the polymer layer is incorporated with PTFE or sodium chloride fine particles permit SVS to release itself slowly.

### Industrial Applicability

As mentioned above, the present invention relates to a stent to be implanted in a duct of a living body. The stent comprises a cylindrical stent main body having an opening on each end and extending in axial direction between the two openings, and a sustained release coating formed on the surface of said stent main body from which a biologically/physiologically active substance is released, and wherein said sustained release coating is composed of a layer of a biologically/physiologically active substance which covers the surface of said stent main body, and a polymer layer which covers said layer of a biologically/physiologically active substance on said layer of a biologically/physiologically active substance, and said layer of a biologically/physiologically active substance contains at least one kind of fat-soluble biologically/physiologically active substance, and said polymer layer contains a polymer miscible with said biologically/physiologically active substance and fine particles equal to or smaller than 5 µm in particle diameter dispersed in the polymer. The stent constructed in this way stably loads the biologically/physiologically active substance on the stent main body without decomposition and degradation. In addition, after being implanted in a lesion, it permits the biologically/physiologically active substance to release itself slowly over a long period of time without rapid release in a short period.

The stent main body may be formed from a metallic material. In this case, the stent will be implanted certainly in the lesion because the metallic material has high strength.

The stent main body may be formed from a polymeric material. In this case, the stent will be easily delivered to the lesion because the polymeric material has good flexibility.

The layer of a biologically/physiologically active substance may be composed solely of a biologically/physiologically active substance. In this case, it is possible to form the layer of biologically/physiologically active substance in a simple way.

The biologically/physiologically active substance may be any of antineoplastic agent, immunosuppressor, antibiotic, antirheumatic, antilipemic agent, ACE inhibitor, calcium antagonist, antiallergic agent, retinoid, antioxidant, and anti-inflammatory agent. Any of these biologically/physiologically active substance prevents restenosis while the stent is implanted in the lesion.

The polymer layer may be formed from a silicone polymer. In this case, the stent exhibits outstanding safety to the living body.

The fine particles may be those of a low-molecular-weight water-soluble salt having a molecular weight not higher than 1000 which is one of the salts present in a living body. In this case, the stent is highly safe because the salt has a low level of stimulus to a living body.

The low-molecular-weight water-soluble salt may be sodium chloride. In this case, the stent is much safer because sodium chloride is present in a high ratio in a living body.

The fine particles may be an inactive substance such as silica filler and PTFE. In this case, such fine particles are inactive to a living body. In addition, it can be made readily dispersible into or miscible with the polymer layer by surface treatment in various ways.

## Claims

1. A stent to be implanted in a duct of a living body, comprising:
a cylindrical stent main body having an opening on each end and extending in axial direction between the two openings and
a sustained release coating formed on the surface of said stent main body from which a biologically/physiologically active substance is released; wherein
said sustained release coating composed of:
a layer of a biologically/physiologically active substance which covers the surface of said stent main body; and
a polymer layer which covers said layer of a biologically/physiologically active substance on said layer of a biologically/physiologically active substance,
said layer of a biologically/physiologically active substance containing at least one kind of fat-soluble biologically/physiologically active substance,
said polymer layer containing a polymer miscible with said biologically/physiologically active substance and fine particles equal to or smaller than 5 µm in particle diameter dispersed in the polymer.

2. The stent as defined in Claim 1, wherein the stent main body is formed from a metallic material.

3. The stent as defined in Claim 1, wherein the stent main body is formed from a polymeric material.

4. The stent as defined in any of Claims 1 to 3, wherein the layer of a biologically/physiologically active substance is composed solely of a biologically/physiologically active substance.

5. The stent as defined in any of Claims 1 to 4, wherein the biologically/physiologically active substance is any of antineoplastic agent, immunosuppressor, antibiotic, antirheumatic, antilipemic agent, ACE inhibitor, calcium antagonist, antiallergic agent, retinoid, antioxidant, and anti-inflammatory agent.

6. The stent as defined in any of Claims 1 to 5, wherein the biologically/physiologically active substance is one which has a molecular weight not higher than 600.

7. The stent as defined in any of Claims 1 to 6, wherein the polymer is a silicone polymer.

8. The stent as defined in any of Claims 1 to 7, wherein the fine particles are those of a low-molecular-weight water-soluble salt having a molecular weight not higher than 1000.

9. The stent as defined in Claim 8, wherein the low-molecular-weight salt is sodium chloride.

10. The stent as defined in any of Claims 1 to 7, wherein the fine particles are those of a water-insoluble substance.

11. The stent as defined in Claim 10, wherein the water-insoluble substance is a silica filler.

12. The stent as defined in Claim 10, wherein the water-insoluble substance is polytetrafluoroethylene.
